# EUROPEAN PATENT APPLICATION

(11) **EP 4 524 840 A1**
(43) Date of publication of application: **19.03.2025**
(21) Application number: 23803514.1
(22) Date of filing: 01.05.2023
(51) Int. Cl.: G06Q 10/04, G16C 60/00

(54) **PREDICTION DEVICE, MATERIAL DESIGN SYSTEM, PREDICTION METHOD, AND PREDICTION PROGRAM**

(30) Priority: 13.05.2022 JP 2022079269
(71) Applicant: Resonac Corporation, Tokyo 105-7325 (JP)
(72) Inventor: MINAMI, Takuya, Tokyo 105-8518 (JP); FUJIMORI, Takahiro, Tokyo 105-8518 (JP); LEE, Haein, Tokyo 105-8518 (JP); OKUNO, Yoshishige, Tokyo 105-8518 (JP)
(74) Representative: Strehl & Partner mbB
(86) International application number: PCT/JP2023/017086
(87) International publication number: WO 2023/219037

(57) **Abstract**

To improve the development efficiency of a new material, a prediction device includes a section determination unit configured to acquire a training data set used for generating a trained prediction model, and determine a plurality of sections for classifying attribute values from a frequency distribution of the attribute values calculated between a plurality of data included in the training data set, an evaluation unit configured to determine sections to which attribute values calculated between prediction target data and the plurality of data are classified into, among the plurality of sections, and evaluate an appropriateness of the prediction target data with respect to conflicting indexes, and a display unit configured to display a predicted value predicted by the trained model in association with an evaluation result of the evaluation unit, by inputting the prediction target data to the trained model.

## Description

### TECHNICAL FIELD

The present disclosure relates to prediction devices, material design systems, prediction methods, and prediction programs.

### BACKGROUND ART

In recent years, when developing new materials, attempts or the like were made to predict a material characteristic using a trained prediction model generated by machine learning, for example. This is because, by using the trained prediction model, it is possible to reduce a number of processes, such as generation of a material, verification experiment of the material characteristic, or the like, and improvement of a development efficiency of the new material can be expected.

On the other hand, in the case of the trained prediction model generated by machine learning, it is known that a prediction accuracy deteriorates if prediction target data (input data) significantly deviates from training data used in the machine learning.

In contrast, the following Non-Patent Document 1 or the like, for example, proposes defining an application range (a range of prediction target data capable of achieving a desired prediction accuracy) of the prediction model.

### PRIOR ART DOCUMENTS

### NON-PATENT DOCUMENTS

Non-Patent Document 1: Igor I. Baskin, Natalia Kireeva, and Alexandre Varnek, "The One-Class Classification Approach to Data Description and to Models Applicability Domain", Nol. Inf. 2010, 29, 581p-587p.

### DISCLOSURE OF THE INVENTION

### PROBLEMS TO BE SOLVED BY THE INVENTION

However, when developing the new material, even if the material characteristic is predicted for the prediction target data close to the training data, the prediction target data lacks in unexpectedness, and a possibility of developing a useful new material is low.

On the other hand, in the case of the prediction target data significantly deviated from the training data, the prediction accuracy is poor to start with, and thus, even if a material characteristic satisfying a predetermined condition is predicted, the material characteristic as predicted is rarely obtained when the material is actually generated, and the possibility of developing a useful new material is also low.

In view of the above, when developing a new material, it may be considered important to define the application range of the prediction model under two conflicting indexes, that is, the unexpectedness and the prediction accuracy, in order to improve the development efficiency.

One object of the present disclosure is to improve a development efficiency of a new material.

### MEANS OF SOLVING THE PROBLEMS

A prediction device according to a first aspect of the present disclosure includes:
a section determination unit configured to acquire a training data set used for generating a trained prediction model, and determine a plurality of sections for classifying attribute values from a frequency distribution of the attribute values calculated between a plurality of data included in the training data set;
an evaluation unit configured to determine sections to which attribute values calculated between prediction target data and the plurality of data are classified into, among the plurality of sections, and evaluate an appropriateness of the prediction target data with respect to conflicting indexes; and
a display unit configured to display a predicted value predicted by the trained model in association with an evaluation result of the evaluation unit, by inputting the prediction target data to the trained model.

According to a second aspect of the present disclosure, in the prediction device according to the first aspect, the section determination unit calculates descriptive statistics for the attribute values calculated between the plurality of data, and determines a lower limit value or an upper limit value of the attribute values that defines the plurality of sections.

According to a third aspect of the present disclosure, in the prediction device according to the second aspect, the section determination unit determines three or more sections that do not overlap one another.

According to a fourth aspect of the present disclosure, in the prediction device according to the third aspect, the evaluation unit evaluates the appropriateness of the prediction target data depending on a closeness of a section to which the attribute value calculated between the prediction target data and the plurality of data is classified into among the three or more sections, with respect to a section including a predetermined descriptive statistic.

According to a fifth aspect of the present disclosure, in the prediction device according to the third aspect, the evaluation unit excludes the prediction target data from data to be input to the trained model when a section to which the attribute value calculated between the prediction target data and the plurality of data is classified into among the three or more sections is determined to be most distant from a section including a predetermined descriptive statistic.

According to a sixth aspect of the present disclosure, in the prediction device according to the third aspect, the evaluation unit selects the prediction target data as the data to be input to the trained model when the attribute value calculated between the prediction target data and the plurality of data is classified into among the three or more sections is determined as a section R-th closest to a section including a predetermined descriptive statistic.

According to a seventh aspect of the present disclosure, the prediction device according to the first aspect further includes:
a training data attribute value calculation unit configured to calculate distances among the plurality of data included in the training data set; and
a training data minimum attribute value extraction unit configured to extract a minimum distance among calculated distances between each of the plurality of data and other data,
wherein the section determination unit determines a plurality of sections for classifying the minimum distance from a frequency distribution of the extracted minimum distance.

According to an eighth aspect of the present disclosure, in the prediction device according to the seventh aspect,
the training data attribute value calculation unit calculates distances between an i-th data (1 <= i <= N) and (N-1) data excluding the i-th data among N data (N is an arbitrary integer) included in the training data set, and
the training data minimum attribute value extraction unit extracts the minimum distance from (N-1) distances calculated for the i-th data.

According to a ninth aspect of the present disclosure, the prediction device according to the seventh aspect further includes:
a prediction target data attribute value calculation unit configured to calculate the distance between the prediction target data and the plurality of data; and
a prediction target data minimum attribute value extraction unit configured to extract a minimum distance among distances between the prediction target data and the plurality of data,
wherein the evaluation unit evaluates the appropriateness of the prediction target data with respect to the conflicting indexes by determining the sections to which the minimum distance extracted for the prediction target data is classified into, among the plurality of sections.

A material design system according to a tenth aspect of the present disclosure includes:
the prediction device according to the first aspect; and
a material design device configured to receive the prediction target data for which the evaluation unit of the prediction device determines that the attribute values calculated between the prediction target data and the plurality of data are to be classified into a predetermined section, and for which the trained model of the prediction device predicts the predicted value satisfying a predetermined condition, and generates material design data.

According to an eleventh aspect of the present disclosure, the material design system according to the tenth aspect further includes:
a training device configured to generate the trained model based on the training data set,
wherein the prediction device predicts the predicted value by inputting the prediction target data to the trained model generated by the training device.

A computer-implemented prediction method according to a twelfth aspect of the present disclosure includes the steps of:
acquiring a training data set used for generating a trained prediction model, and determining a plurality of sections for classifying attribute values from a frequency distribution of the attribute values calculated between a plurality of data included in the training data set;
determining sections to which attribute values calculated between prediction target data and the plurality of data are classified into, among the plurality of sections, and evaluating an appropriateness of the prediction target data with respect to conflicting indexes; and
displaying a predicted value predicted by the trained model in association with an evaluation result of the determining step, by inputting the prediction target data to the trained model.

A prediction program according to a thirteenth aspect of the present disclosure causes a computer to execute the steps of:
acquiring a training data set used for generating a trained prediction model, and determining a plurality of sections for classifying attribute values from a frequency distribution of the attribute values calculated between a plurality of data included in the training data set;
determining sections to which attribute values calculated between prediction target data and the plurality of data are classified into, among the plurality of sections, and evaluating an appropriateness of the prediction target data with respect to conflicting indexes; and
displaying a predicted value predicted by the trained model in association with an evaluation result of the determining step, by inputting the prediction target data to the trained model.

### EFFECTS OF THE INVENTION

According to the present disclosure, it is possible to improve the development efficiency of the new material.

### BRIEF DESCRIPTION OF THE DRAWINGS

[FIG. 1] FIG. 1 is a diagram illustrating an example of a system configuration of a material design system.
[FIG. 2] FIG. 2 is a diagram illustrating an example of functional configurations of a training device and a prediction device.
[FIG. 3] FIG. 3 is a diagram illustrating an example of a hardware configuration of the training device and the prediction device.
[FIG. 4] FIG. 4 is a diagram illustrating a specific example of processes of a training unit of the training device.
[FIG. 5] FIG. 5 is a diagram illustrating a specific example of processes of a training data attribute value calculation unit of the prediction device.
[FIG. 6] FIG. 6 is a diagram illustrating a specific example of processes of a section determination unit of the prediction device.
[FIG. 7] FIG. 7 is a diagram illustrating a specific example of processes of a prediction target data attribute value calculation unit of the prediction device.
[FIG. 8] FIG. 8 is a diagram illustrating a specific example of processes of an evaluation unit of the prediction device.
[FIG. 9] FIG. 9 is a diagram illustrating a specific example of processes of a prediction unit of the prediction device.
[FIG. 10] FIG. 10 is a diagram illustrating a specific example of processes of a display unit of the prediction device.
[FIG. 11] FIG. 11 is a flow chart illustrating a flow of a training process.
[FIG. 12] FIG. 12 is a flow chart illustrating a flow of a prediction process.
[FIG. 13] FIG. 13 is a diagram illustrating an exemplary implementation 1.
[FIG. 14] FIG. 14 is a diagram illustrating an exemplary implementation **2.**
[FIG. 15] FIG. 15 is a diagram illustrating an exemplary implementation 3.
[FIG. 16] FIG. 16 is a diagram illustrating verification examples of predicted characteristics.

### MODE OF CARRYING OUT THE INVENTION

Hereinafter, each embodiment will be described with reference to the accompanying drawings. In the present specification and drawings, constituent elements having substantially the same functional configuration are designated by the same reference numerals, and a redundant description thereof will be omitted.

### [First Embodiment]

### <System Configuration of Material Design System>

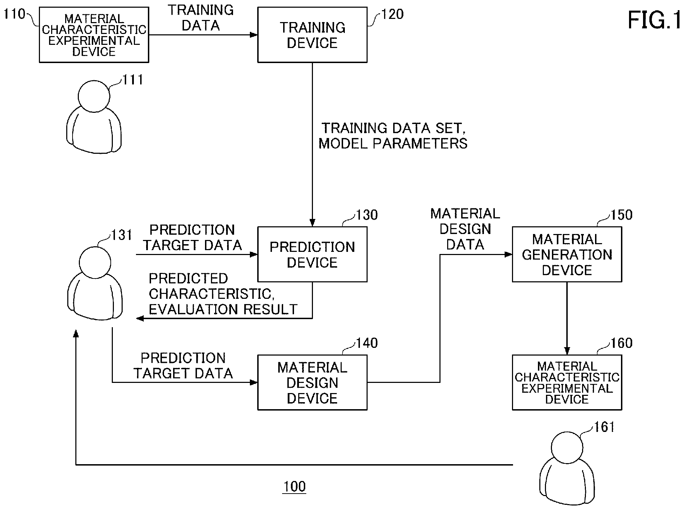

First, a system configuration of a material design system according to a first embodiment will be described. FIG. 1 is a diagram illustrating an example of the system configuration of the material design system. As illustrated in FIG. 1, a material design system 100 includes a material characteristic experimental device 110, a training device 120, a prediction device 130, a material design device 140, a material generation device 150, and a material characteristic experimental device 160.

The material characteristic experimental device 110 is a device configured to obtain, through experimentation, a characteristic value of "a material characteristic" of a material described by "material data" that is a known structural formula. The characteristic value of each material obtained by an experimenter 111 using the material characteristic experimental device 110 is input to the training device 120 as training data, together with a corresponding structural formula. The training data may be configured based on information of a known database.

The training device 120 performs a machine learning on a prediction model (a model for predicting the characteristic value of the material from the structural formula of the material), using a training data set including a plurality of acquired training data, and generates a trained prediction model. In addition, the training device 120 notifies the training data set used to generate the trained prediction model, and model parameters of the trained prediction model, to the prediction device 130.

The prediction device 130 includes a trained prediction model to which the model parameters notified from the training device 120 are set, and predicts predicted characteristic (an example of prediction value) of prediction target data input by a designer 131.

Moreover, the prediction device 130 analyzes the training data set notified from the training device 120, and quantitatively calculates a degree of deviation of the material data of each training data from material data of other training data, so as to calculate attribute values between the material data.

Further, the prediction device 130 calculates a frequency distribution of the attribute values between the calculated material data, so as to determine a plurality of sections for sectioning the attribute values (specifically, a lower limit value and an upper limit value of the attribute values defining each section).

In addition, the prediction device 130 quantitatively calculates the degree of deviation of the material data of the prediction target data from the material data of each training data, so as to calculate the attribute values of the material data of the prediction target data. Furthermore, the prediction device 130 determines which of the determined plurality of sections the attribute values of the material data of the prediction target data are sectioned, to evaluate an appropriateness of the prediction target data, and outputs an evaluation result in association with the predicted characteristic.

The appropriateness of the prediction target data refers to an appropriateness of the prediction target data with respect to two conflicting indexes, that is, unexpectedness and prediction accuracy during development of a new material. The high appropriateness of the prediction target data indicates that the unexpectedness of the prediction target data with respect to the training data, and the prediction accuracy regarding the predicted characteristic of the prediction target data, are balanced (in the present embodiment, the appropriateness is represented as a "rank" (details will be described later)).

The predicted characteristic of the prediction target data and the evaluation result of the prediction target data output by the prediction device 130 are notified to the designer 131. Accordingly, the designer 131 can grasp the predicted characteristic of the prediction target data, and can also grasp whether or not the material generated based on the prediction target data has a high possibility of becoming a useful new material.

The material design device 140 is a device configured to generate material design data. The designer 131 selects the prediction target data having a high possibility of becoming a useful new material, and inputs the prediction target data to the material design device 140, so as to generate the material design data.

The material generation device 150 is a device configured to actually generate the material, based on the generated material design data.

The material characteristic experimental device 160 is a device configured to obtain, through verification experimentation, the material characteristic of the new material that is actually generated by the material generation device 150. The material characteristic of the new material obtained by an experimenter 161 using the material characteristic experimental device 160 is notified to the designer 131.

### <Functional Configurations of Training Device and Prediction Device>

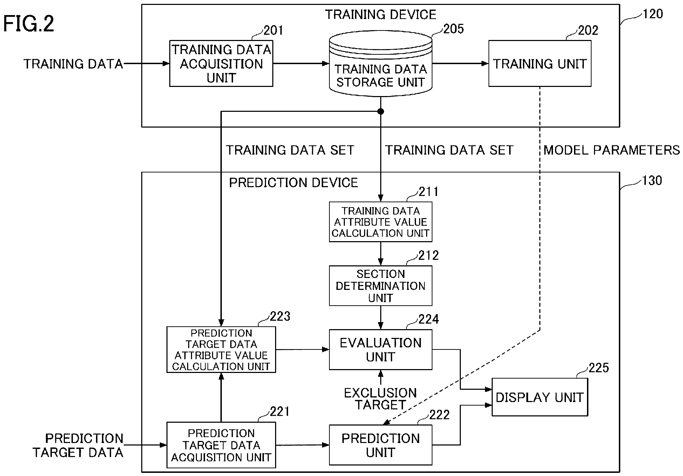

Next, functional configurations of the training device 120 and the prediction device 130, among the devices configuring the material design system 100, will be described with reference to FIG. 2. FIG. 2 is a diagram illustrating an example of the functional configurations of the training device and the prediction device.

A training program is installed in the training device 120, and the training device 120 functions as a training data acquisition unit 201 and a training unit 202 by executing the training program.

The training data acquisition unit 201 acquires training data input by the experimenter 111, and stores the training data in a training data storage unit 205.

The training unit 202 performs machine learning on the prediction model, using a training data set including a plurality of training data stored in the training data storage unit 205, and generates a trained prediction model. The model parameters of the trained prediction model generated by the training unit 202 are notified to the prediction device 130, together with the training data set used for generating the trained prediction model.

A prediction program is installed in the prediction device 130. The prediction device 130 functions as a training data attribute value calculation unit 211, a section determination unit 212, a prediction target data acquisition unit 221, a prediction unit 222, a prediction target data attribute value calculation unit 223, an evaluation unit 224, and a display unit 225 by executing the prediction program.

The training data attribute value calculation unit 211 analyzes the training data set notified from the training device 120, and quantitatively calculates the degree of deviation of the material data of each training data from the material data of other training data, so as to calculate the attribute values between the material data.

The section determination unit 212 calculates the frequency distribution of the attribute values between the material data calculated by the training data attribute value calculation unit 211, so as to determine the plurality of sections for sectioning the attribute values (specifically, the lower limit value and the upper limit value of the attribute values defining each section).

The prediction target data acquisition unit 221 acquires the prediction target data input by the designer 131, and notifies the prediction target data to the prediction unit 222 and the prediction target data attribute value calculation unit 223.

The prediction unit 222 has the trained prediction model to which the model parameters notified from the training unit 202 are set, and predicts the predicted characteristic of the prediction target data input by the designer 131.

The prediction target data attribute value calculation unit 223 acquires each training data of the training data set notified from the training device 120, and also acquires the prediction target data notified from the prediction target data acquisition unit 221.

In addition, the prediction target data attribute value calculation unit 223 quantitatively calculates the degree of deviation of the material data of the prediction target data from the material data of each training data, so as to calculate the attribute values of the material data of the prediction target data.

The evaluation unit 224 determines the sections to which the attribute values of the material data of the prediction target data calculated by the prediction target data attribute value calculation unit 223 are classified into, among the plurality of sections determined by the section determination unit 212. Hence, the evaluation unit 224 evaluates the appropriateness of the prediction target data, and notifies the evaluation result to the display unit 225.

An exclusion target is set in advance in the evaluation unit 224, and in a case where the calculated attribute value is classified into a predetermined section, the prediction target data is excluded from the prediction target of the prediction unit 222 (that is, the prediction target data will not be input to the prediction model trained by the prediction unit 222). Alternatively, in the case where the calculated attribute value is classified into the predetermined section, the evaluation unit 224 excludes the prediction target data from the display target of the display unit 225 (that is, the prediction target data will not be displayed by the display unit 225).

The display unit 225 displays the predicted characteristic of the prediction target data predicted by the prediction unit 222, and the evaluation result of the prediction target data evaluated by the evaluation unit 224, in association with each other.

Accordingly, the material design system 100 according to the first embodiment includes:
· The prediction device 130 configured to predict the predicted characteristic of the prediction target data. Thus, according to the first embodiment, the prediction target data for which the predicted characteristic satisfying the predetermined condition is not predicted can be excluded from the target of the material generation and the verification experiment.
· The prediction device 130 configured to output the evaluation result of the prediction target data. Thus, according to the first embodiment, even in a case where the prediction device 130 predicts the predicted characteristic satisfying the predetermined condition, the prediction target data having a low possibility of becoming a useful new material can be excluded from the target of the material generation and the verification experiment.

As a result, the material design system 100 according to the first embodiment can narrow down the prediction target data for which the material generation and the verification experiment are to be performed, and can improve the development efficiency of the new material.

### <Hardware Configuration of Training Device and Prediction Device>

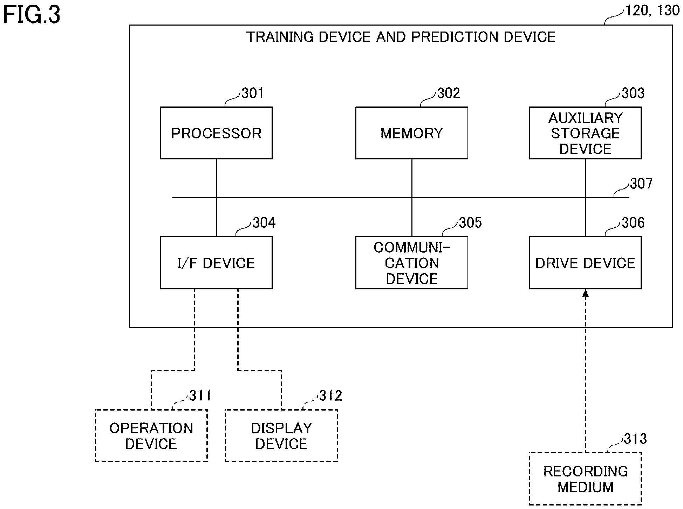

Next, a hardware configuration of the training device 120 and the prediction device 130 will be described. Because the training device 120 and the prediction device 130 have the same hardware configuration, the hardware configuration of the training device 120 and the prediction device 130 will be collectively described in this example, with reference to FIG. 3.

FIG. 3 is a diagram illustrating an example of a hardware configuration of the training device and the prediction device. As illustrated in FIG. 3, the training device 120 and the prediction device 130 include a processor 301, a memory 302, an auxiliary storage device 303, an interface (I/F) device 304, a communication device 305, and a drive device 306. The hardware of the training device 120 and the hardware of the prediction device 130 are connected to each other via a bus 307.

The processor 301 includes various computing devices, such as a central processing unit (CPU), a graphics processing unit (GPU), or the like. The processor 301 reads various programs (for example, a training program, a prediction program, or the like) into the memory 302 and executes the programs.

The memory 302 includes a main storage device, such as a read only memory (ROM), a random access memory (RAM), or the like. The processor 301 and the memory 302 form a so-called computer, and the processor 301 executes the various programs read into the memory 302, such that the computer implements the various functions described above.

The auxiliary storage device 303 stores various programs and various data used when the various programs are executed by the processor 301. For example, the training data storage unit 205 is implemented in the auxiliary storage device 303.

The I/F device 304 is a connection device configured to connect to an operation device 311 and a display device 312, which are examples of a user interface device. The communication device 305 is a communication device configured to communicate with an external device (not illustrated) via a network.

The drive device 306 is a device configured to receive a recording medium 313 that is set therein. The recording medium 313 includes a medium for optically, electrically, or magnetically recording information, such as a CD-ROM, a flexible disk, a magneto-optical disk, or the like. The recording medium 313 may include a semiconductor memory or the like that electrically records information, such as a ROM, a flash memory, or the like.

The various programs installed in the auxiliary storage device 303 are installed by setting a distributed recording medium 313 in the drive device 306, and reading the various programs recorded in the recording medium 313 by the drive device 306, for example. Alternatively, the various programs installed in the auxiliary storage device 303 may be installed by being downloaded from the network via the communication device 305.

### <Specific Example of Processes of Each Unit of Training Device>

Next, a specific example of processes of each unit (the training unit 202 in this example) of the training device 120 will be described.

### (1) Specific Example of Processes of Training Unit 202:

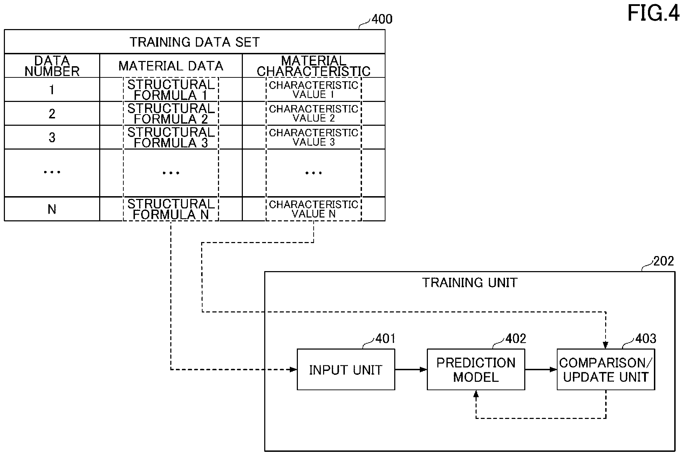

First, a specific example of the processes performed by the training unit 202 of the training device 120 will be described. FIG. 4 is a diagram illustrating a specific example of the processes of the training unit of the training device. As illustrated in FIG. 4, the training unit 202 includes an input unit 401, a prediction model 402, and a comparison/update unit 403.

In FIG. 4, a training data set 400 includes "data number", "material data", and "material characteristic", as items of information. The "data number" stores a number for identifying each training data, the "material data" stores the structural formula of each material, and the "material characteristic" stores the characteristic value of each material. The example of FIG. 4 illustrates a case where N training data (N is an arbitrary integer) are stored.

The input unit 401 reads the structural formula of the material included in the "material data" of each training data from the training data set 400, and inputs the structural formula to the prediction model.

The prediction model 402 outputs output data corresponding to the material characteristic, by inputting the structural formula of the material included in the "material data" of each training data.

The comparison/update unit 403 updates model parameters of the prediction model 402, such that the output data output from the prediction model 402 compared therewith approaches the characteristic value of the material included in the "material characteristic" of each training data.

Accordingly, the training unit 202 can generate a trained prediction model capable of predicting the material characteristic of the prediction target data, based on the material data of the prediction target data.

### <Specific Example of Processes of Each Unit of Prediction Device>

Next, a specific example of processes performed by each unit (the training data attribute value calculation unit 211, the section determination unit 212, the prediction target data attribute value calculation unit 223, the evaluation unit 224, the prediction unit 222, and the display unit 225 in this example) of the prediction device 130 will be described.

### (1) Specific Example of Processes of Training Data Attribute Value Calculation Unit 211:

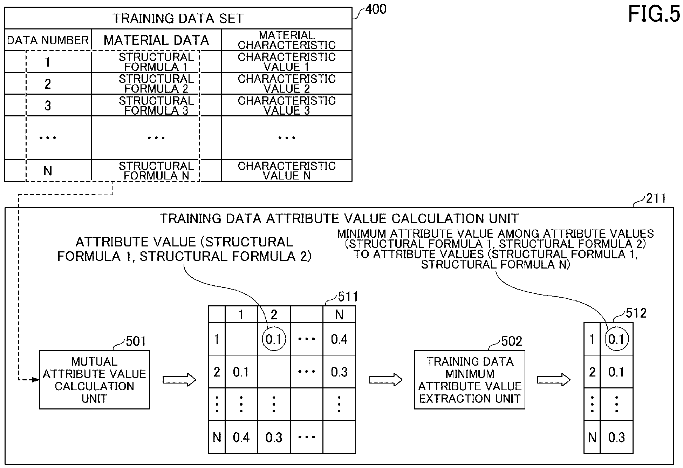

First, a specific example of the processes performed by the training data attribute value calculation unit 211 will be described. FIG. 5 is a diagram illustrating a specific example of processes of the training data attribute value calculation unit of the prediction device. As illustrated in FIG. 5, the training data attribute value calculation unit 211 includes a mutual attribute value calculation unit 501 and a training data minimum attribute value extraction unit 502.

The mutual attribute value calculation unit 501 reads the number for identifying each training data and the structural formula of each material from the "data number" and the "material data" of the training data set 400 including N training data, respectively.

In addition, the mutual attribute value calculation unit 501 generates, from the structural formulae of the materials included in the "material data" of the N training data, combinations of:
· A structural formula of a material included in the "material data" of i-th (1 <= i <= N) training data, and
· Structural formulae of materials included in the "material data" of other (N-1) training data excluding the i-th training data,
and calculates the attribute value between the material data for all of the combinations. The attribute value between the material data referred above indicates a value indicating a degree of deviation between the structural formula of the material included in the "material data" of the i-th training data and the structural formula of the material included in the "material data" of the training data other than the i-th training data. Specifically, the attribute value between the material data refers to a distance between the structural formula of the material included in the "material data" of the i-th training data and the structural formula of the material included in the "material data" of the training data other than the i-th training data. Alternatively, the attribute value between the material data may refer to a density or the like between the structural formula of the material included in the "material data" of the i-th training data and the structural formula of the material included in the "material data" of the training data other than the i-th training data.

In FIG. 5, a table 511 is a table having numbers for identifying the training data are arranged along the abscissa and the ordinate. The table 511 illustrates all combinations of the material data of each training data included in the training data set 400.

In addition, in a region where the number indicated along the abscissa and the number indicated along the ordinate intersect in the table 511, the attribute value between the material data calculated for the combination of the material data of the corresponding training data is indicated.

For example, an attribute value (= 0.1) between the material data calculated for the combination of:
· The material data = "structural formula 2" of the training data having the data number = "2", and
· The material data = "structural formula "1" of the training data having the data number = "1",
is stored in the region where the data number = "2" along the abscissa and the data number = "1" along the ordinate intersect.

The training data minimum attribute value extraction unit 502 extracts a minimum attribute value (for example, a minimum distance) for each material, from the attribute value between the material data for all combinations calculated by the mutual attribute value calculation unit 501.

In FIG. 5, a table 512 illustrates the minimum attribute value extracted for each material by the training data minimum attribute value extraction unit 502 in association with the number for identifying each training data.

For example, in the case of the training data identified by the data number = "1", the minimum attribute value of the material is "0.1".

The minimum attribute value of each material extracted by the training data minimum attribute value extraction unit 502 is notified to the section determination unit 212.

### (2) Specific Example of Processes of Section Determination Unit 212

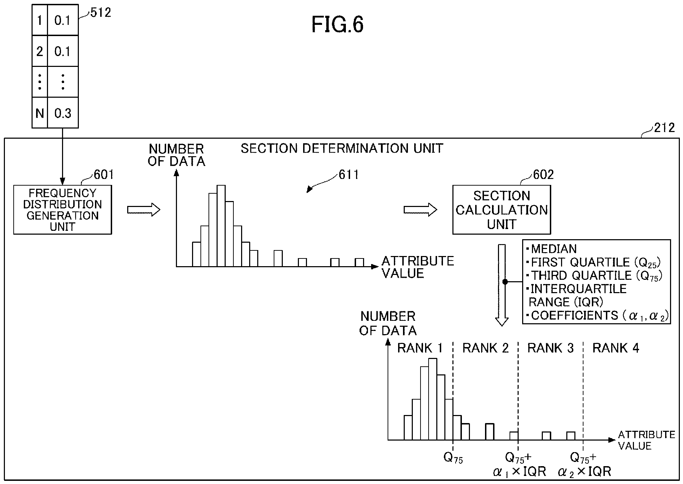

Next, a specific example of the processes performed by the section determination unit 212 will be described. FIG. 6 is a diagram illustrating a specific example of the processes of the section determination unit of the prediction device. As illustrated in FIG. 6, the section determination unit 212 includes a frequency distribution generation unit 601 and a section calculation unit 602.

The frequency distribution generation unit 601 acquires the minimum attribute value of each material notified from the training data minimum attribute value extraction unit 502, and generates a frequency distribution. In FIG. 6, a reference numeral 611 denotes an example of the frequency distribution generated by the frequency distribution generation unit 601, the abscissa represents the attribute value, and the ordinate represents the number of data.

The section calculation unit 602 calculates descriptive statistics, based on the minimum attribute value of each material acquired by the frequency distribution generation unit 601. Specifically, the section calculation unit 602 calculates:
· A median of the minimum attribute value of each material,
· A first quartile (Q₂₅) of the minimum attribute value of each material,
· A third quartile (Q₇₅) of the minimum attribute value of each material, and
· An interquartile range (IQR = Q₇₅ - Q₂₅) of the minimum attribute value of each material.
In addition, the section calculation unit 602 determines coefficients (α₁, α₂) that are used when determining the plurality of sections for classifying each attribute value, based on the calculated descriptive statistics.

In the example of FIG. 6, the coefficient α₁ = "1.5" and the coefficient α₂ = "3.0" are determined, and FIG. 6 illustrates a state where:
· An upper limit value of a section in which an attribute value satisfying "attribute value < (third quartile (Q₇₅))" is classified,
· A lower limit value and the upper limit value of the section in which the attribute value satisfying "(third quartile (Q₇₅)) <= attribute value < (value obtained by adding value obtained by multiplying α₁ to interquartile range (IQR)) to third quartile (Q₇₅)" is classified,
· The lower limit value and the upper limit value of the section in which the attribute value satisfying "(value obtained by adding value obtained by multiplying α₁ to interquartile range (IQR) to third quartile (Q₇₅)) <= attribute value < (value obtained by adding value obtained by multiplying α₂ to interquartile range (IQR) to third quartile (Q₇₅))" is classified, and
· The lower limit value of the section in which the attribute value satisfying "(value obtained by adding value obtained by multiplying α₂ to interquartile range (IQR) to third quartile (Q₇₅)) <= attribute value" is classified,
with respect to the frequency distribution (reference numeral 611) generated by the frequency distribution generation unit 601.

The example of FIG. 6 defines:
· A section including a predetermined descriptive statistic ("median" in this example) as "rank 1",
· A section first closest to the section including the predetermined descriptive statistic as "rank 2",
· A section second closest to the section including the predetermined descriptive statistic as "rank 3", and
· A section most distant from the section including the predetermined descriptive statistic as "rank 4".

Next, when a relationship between the rank described above and the two conflicting indexes (unexpectedness and prediction accuracy) when developing a new material are taken into consideration:
· The prediction accuracy decreases in the order of "rank 1" → "rank 2" → "rank 3" → "rank 4". That is, the rank represents how high the prediction accuracy is.
· The unexpectedness increases in the order of "rank 1" → "rank 2" → "rank 3" → "rank 4". That is, the rank represents how low the unexpectedness is.

Accordingly, it may be regarded that each rank simultaneously represents the two conflicting indexes (unexpectedness and prediction accuracy) when developing the new material.

### (3) Specific Example of Processes of Prediction Target Data Attribute Value Calculation Unit 223:

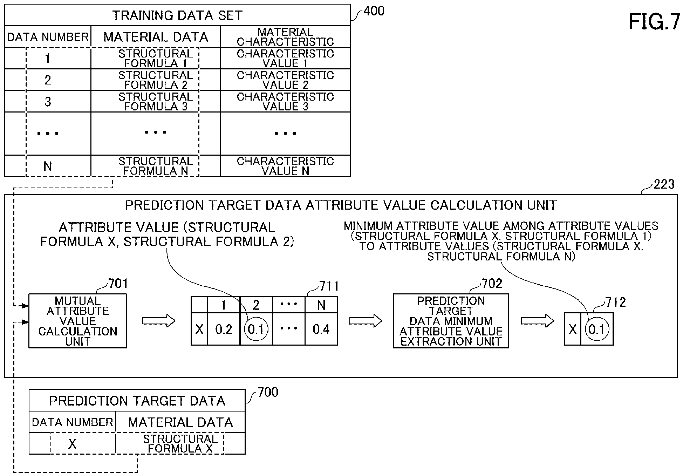

Next, a specific example of the processes performed by the prediction target data attribute value calculation unit 223 will be described. FIG. 7 is a diagram illustrating a specific example of the processes of the prediction target data attribute value calculation unit of the prediction device. As illustrated in FIG. 7, the prediction target data attribute value calculation unit 223 includes a mutual attribute value calculation unit 701 and a prediction target data minimum attribute value extraction unit 702.

The mutual attribute value calculation unit 701 reads the number for identifying each training data and the structural formula of each material, from the "data number" and the "material data" of the training data set 400 including the N training data notified from the training device 120, respectively. In addition, the mutual attribute value calculation unit 701 reads the number for identifying the prediction target data and the structural formula of the material, from the "data number" and the "material data" of prediction target data 700 notified from the prediction target data acquisition unit 221.

Further, the mutual attribute value calculation unit 701 generates, from the structural formulae of the materials included in the "material data" of the read N training data, combinations of:
· A structural formula of the material included in the "material data" of i-th (1 <= i <= N) training data, and
· Structural formulae of the materials included in the "material data" of the prediction target data 700,
and calculates the attribute value between the material data for all combinations. The attribute value between the material data referred above indicates a value indicating a degree of deviation between the structural formula of the material included in the "material data" of the i-th training data and the structural formula of the material included in the "material data" of the prediction target data 700. Specifically, the attribute value between the material data refers to a distance between the structural formula of the material included in the "material data" of the i-th training data and the structural formula of the material included in the "material data" of the prediction target data 700. Alternatively, the attribute value between the material data may refer to a density or the like between the structural formula of the material included in the "material data" of the i-th training data and the structural formula of the material included in the "material data" of the prediction target data 700.

In FIG. 7, a table 711 is a table having numbers for identifying the training data included in the training data set 400 arranged along the abscissa and numbers for identifying the prediction target data arranged along the ordinate. The table 711 illustrates all combinations of the material data of each training data included in the training data set 400 and the material data of the prediction target data.

In addition, in a region where the number indicated along the abscissa and the number indicated along the ordinate intersect in the table 711, the attribute value between the material data calculated for the combination of the material data of the corresponding training data and the material data of the corresponding prediction target data is indicated.

For example, an attribute value (= 0.1) between the material data calculated for the combination of:
· The material data = "structural formula 2" of the training data having the data number = "2", and
· The material data = "structural formula "X" of the prediction target data having the data number = "X", is stored in the region where the data number = "2" along the abscissa and the data number = "X" along the ordinate intersect.

The prediction target data minimum attribute value extraction unit 702 extracts the minimum attribute value from the attribute value between the material data for all combinations calculated by the mutual attribute value calculation unit 701.

In FIG. 7, a table 712 illustrates the minimum attribute value extracted by the prediction target data minimum attribute value extraction unit 702 in association with the number for identifying the prediction target data. The example of FIG. 7 indicates that the minimum attribute value is "0.1".

The minimum attribute value extracted by the prediction target data minimum attribute value extraction unit 702 is notified to the evaluation unit 224.

### (4) Specific Example of Processes of Evaluation Unit 224:

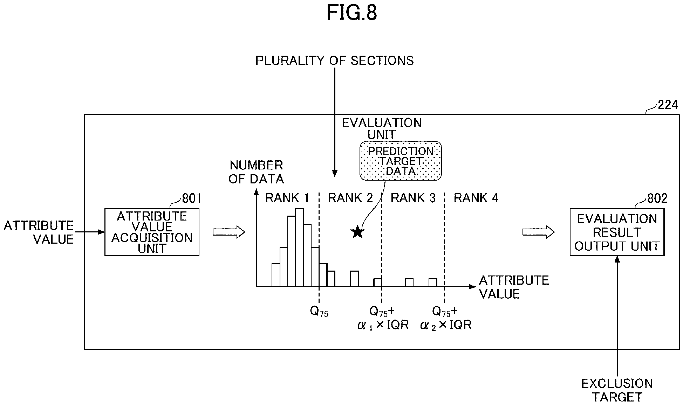

Next, a specific example of the processes performed by the evaluation unit 224 will be described. FIG. 8 is a diagram illustrating a specific example of processes of the evaluation unit of the prediction device. As illustrated in FIG. 8, the evaluation unit 224 includes an attribute value acquisition unit 801 and an evaluation result output unit 802.

The attribute value acquisition unit 801 acquires the minimum attribute value notified from the prediction target data minimum attribute value extraction unit 702, and determines the section to which the minimum attribute value is to be classified into, among the plurality of sections notified from the section determination unit 212. The example of FIG. 8 illustrates a state where it is determined that the minimum attribute value calculated for the structural formula (structural formula X) of the material included in the "material data" of the prediction target data 700 is classified into a section R-th (R = 2) closest to the section including the predetermined descriptive statistic (median value).

Because the section determined by the attribute value acquisition unit 801 is the section R-th (R = 2) closest to the section including the predetermined descriptive statistic, the evaluation result output unit 802 determines that:
· The unexpectedness is the R-th lowest (R = 2), and
· The prediction accuracy is the R-th highest (R = 2), and the appropriateness of the structural formula (structural formula X) of the material included in the "material data" of the prediction target data 700 when developing the new material is (rank 2). That is, the evaluation result output unit 802 evaluates the appropriateness (rank) of the prediction target data, depending on the closeness of the section to which the attribute value of the material data of the prediction target data is classified into, with respect to the section including the predetermined descriptive statistic (median value).

In addition, the evaluation result output unit 802 notifies the evaluation result (rank) to the display unit 225.

Further, the evaluation result output unit 802 determines whether or not the minimum attribute value calculated for the structural formula (structural formula X) of the material included in the "material data" of the prediction target data 700 is classified into a predetermined section. Specifically, the evaluation result output unit 802 determines whether or not the evaluation result (rank) of the appropriateness of the structural formula (structural formula X) of the material included in the "material data" of the prediction target data 700 corresponds to the exclusion target. In a case where the evaluation result is determined as corresponding to the exclusion target, the evaluation result output unit 802 notifies the evaluation result corresponding to the exclusion target to the prediction unit 222 and/or the display unit 225.

### (5) Specific Example of Processes of Prediction Unit 222:

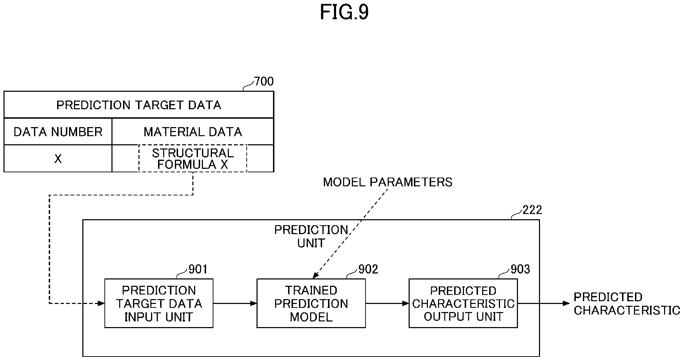

Next, a specific example of the processes performed by the prediction unit 222 will be described. FIG. 9 is a diagram illustrating a specific example of the processes of the prediction unit of the prediction device. As illustrated in FIG. 9, the prediction unit 222 includes a prediction target data input unit 901, a trained prediction model 902, and a predicted characteristic output unit 903.

When the prediction target data 700 is notified from the prediction target data acquisition unit 221, the prediction target data input unit 901 inputs the structural formula (structural formula X) of the material included in the "material data" of the prediction target data 700 to the trained prediction model 902.

In a case where the evaluation result corresponding to the exclusion target is notified from the evaluation result output unit 802, the prediction target data input unit 901 may perform a control so as not to input the structural formula (structural formula X) of the material included in the "material data" of the prediction target data 700 to the trained prediction model 902.

The trained prediction model 902 is a trained prediction model set with the model parameters calculated by performing a training process by the training unit 202. When the structural formula (structural formula X) of the material included in the "material data" of the prediction target data 700 is input, the trained prediction model 902 predicts the predicted characteristic of the prediction target data 700.

The predicted characteristic output unit 903 notifies the predicted characteristic of the prediction target data 700 predicted by the trained prediction model 902 to the display unit 225.

### (6) Specific Example of Processes of Display Unit 225:

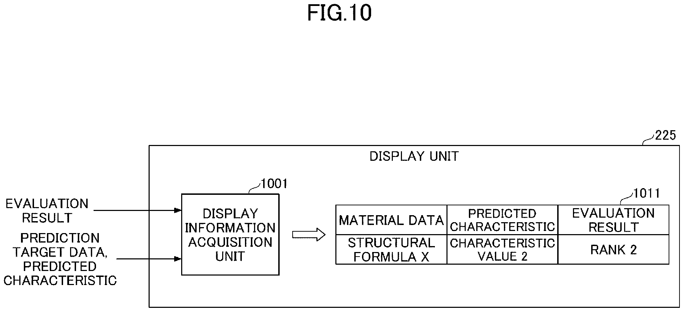

Next, a specific example of processes performed by the display unit 225 will be described. FIG. 10 is a diagram illustrating a specific example of the processes of the display unit of the prediction device. As illustrated in FIG. 10, the display unit 225 includes a display information acquisition unit 1001. The display information acquisition unit 1001 acquires the evaluation result notified from the evaluation unit 224 and the predicted characteristic notified from the prediction unit 222, and generates display data 1011.

As illustrated in FIG. 10, the display data 1011 includes "material data", "predicted characteristic", and "evaluation result", as information items. The "structural formula X", which is the structural formula of the material included in the "material data" of the prediction target data 700, is stored in the "material data", and a "characteristic value 2", which is the predicted characteristic predicted for the prediction target data 700, is stored in the "predicted characteristic". In addition, a "rank 2", which is the evaluation result of the evaluated appropriateness of the structural formula (structural formula X) of the material included in "material data" of the prediction target data 700 when developing the new material, is stored in the "evaluation result".

### <Flow of Training Process by Training Device>

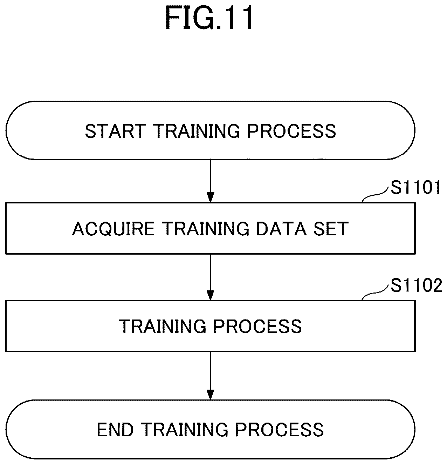

Next, a flow of the training process by the training device 120 will be described. FIG. 11 is a flow chart illustrating the flow of the training process.

In step S1101, the training device 120 acquires the training data set including the material characteristic of each material obtained by an experiment performed by the experimenter 111 using the material characteristic experimental device 110.

In step S1102, the training device 120 performs the training process on the prediction model using the training data set, and generates the trained prediction model.

### <Flow of Prediction Processing by Prediction Device>

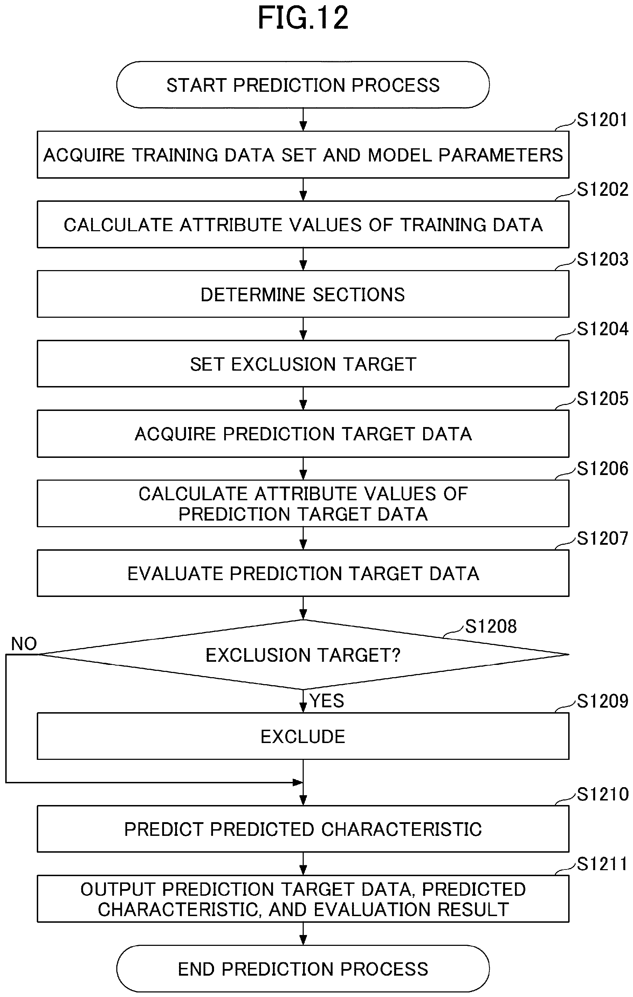

Next, a flow of a prediction process by the prediction device 130 will be described. FIG. 12 is a flow chart illustrating the flow of the prediction process.

In step S1201, the prediction device 130 acquires the training data set used by the training device 120 when generating the trained prediction model, and the model parameters of the trained prediction model.

In step S1202, the prediction device 130 analyzes the training data set, and calculates the attribute values between the material data.

In step S1203, the prediction device 130 calculates the frequency distribution of the calculated attribute values between the material data, and determines the plurality of sections for classifying the attribute values.

In step S1204, the prediction device 130 refers to the setting of the exclusion target.

In step S1205, the prediction device 130 acquires the prediction target data.

In step S1206, the prediction device 130 calculates the attribute values of the material data of the prediction target data.

In step S1207, the prediction device 130 evaluates the appropriateness (rank) of the prediction target data with respect to the two conflicting indexes, by determining the sections to which the calculated attribute values of the material data of the prediction target data are classified into, among the determined plurality of sections.

In step S1208, the prediction device 130 determines whether or not the evaluation result corresponds to the exclusion target. When it is determined in step S1208 that the evaluation result does not correspond to the exclusion target (in a case where a determination result in step S1208 is NO), the process advances to step S1211.

On the other hand, when it is determined in step S1208 that the evaluation result is the exclusion target (in a case where the determination result in step S1208 is YES), the process advances to step S1210.

In step S1209, the prediction device 130 excludes the prediction target data from the prediction target or the display target.

In step S1210, the prediction device 130 predicts the predicted characteristics by inputting the prediction target data to the trained prediction model.

In step S1211, the prediction device 130 displays display data in which the prediction target data, the predicted characteristics, and the evaluation result are associated with one another.

### <Relationship Between Index and Evaluation Result in Each Exemplary Implementation>

Next, results (the predicted characteristics and the evaluation result) of the prediction process performed on a plurality of sets of prediction target data using the prediction device 130 will be illustrated below, and a relationship between the two conflicting indexes and the evaluation result, and a verification example of the predicted characteristics will be described.

Specifically, 100 molecules of a water solubility data set disclosed in a Non-Patent Document which will be described later were learned as a training data set, and remaining unlearned molecules are used as the prediction target data, to predict the water solubility and obtain the evaluation result. The results of three runs a procedure by modifying the molecules of the training data set are described below as exemplary implementations 1 through 3.

The procedure calculates a descriptor from a Simplified Molecular Line Entry System (SMILES) indicating a molecular structure using RDKIT, and uses a value obtained by normalizing the calculated descriptor as an explanatory variable. Ridge regression was used as the prediction model. The normalization and ridge regression are performed by scikit-learn.

### (1) Exemplary Implementation 1:

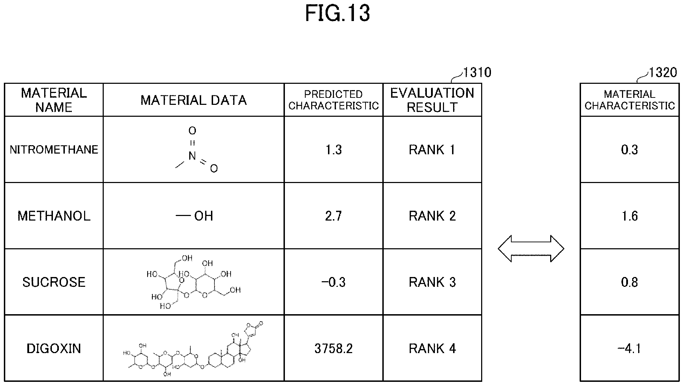

FIG. 13 is a diagram illustrating the exemplary implementation 1. As illustrated in FIG. 13, the exemplary implementation 1 predicts the predicted characteristics of a plurality of prediction target data which are:
· Nitromethane,
· Methanol,
· Sucrose, and
· Digoxin,
and determines the section to which the attribute values were classified into, respectively, to evaluate the appropriateness (refer to a table 1310). Because the material characteristics (the water solubility in this example) of the prediction target data described above are all known, the material characteristics are illustrated in a table 1320 for the purpose of verifying the prediction accuracy.

According to the table 1310, the relationship between the index and the evaluation result for each prediction target data is as follows:
· With respect to the prediction target data evaluated as having the rank 1 and the rank 2, the predicted characteristics with a high water solubility and a good prediction accuracy are obtained, but the structural formula is simple and the unexpectedness is low.
· With respect to the prediction target data evaluated as having the rank 3, the predicted characteristics with a relatively high water solubility and a good prediction accuracy are obtained, and the structural formula is relatively complex and the unexpectedness is high.
· With respect to the prediction target data evaluated as having the rank 4, the predicted characteristics with a very high water solubility are obtained, but the material actually has a low water solubility, and the prediction accuracy is very low. The structural formula is complex and the unexpectedness is high.

### (2) Exemplary Implementation 2:

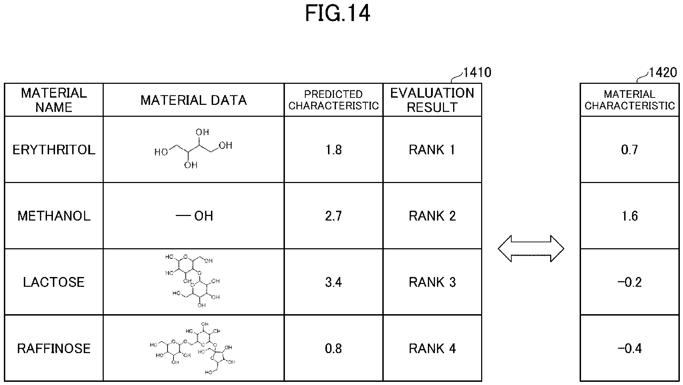

FIG. 14 is a diagram illustrating an exemplary implementation 2. As illustrated in FIG. 14, the exemplary implementation 2 predicts the predicted characteristics of a plurality of prediction target data which are:
· Erythritol,
· Methanol,
· Lactose, and
· Raffinose,
and determines the section to which the attribute values were classified into, respectively, to evaluate the appropriateness (refer to a table 1410). Because the material characteristics (the water solubility also in this example) of the prediction target data described above are all known, the material characteristics are illustrated in a table 1420 for the purpose of verifying the prediction accuracy.

According to the table 1410, the relationship between the index and the evaluation result for each prediction target data is as follows:
· With respect to the prediction target data evaluated as having the rank 1 and the rank 2, the predicted characteristics with a high water solubility and a good prediction accuracy are obtained, but the structural formula is simple and the unexpectedness is low.
· With respect to the prediction target data evaluated as having the rank 3, the predicted characteristics with a relatively high water solubility and a good prediction accuracy are obtained, and the structural formula is relatively complex and the unexpectedness is high.
· With respect to the prediction target data evaluated as having the rank 4, the predicted characteristics with a relatively high water solubility and a good prediction accuracy are obtained, the structural formula is complex and the unexpectedness is high.

### (3) Exemplary Implementation 3:

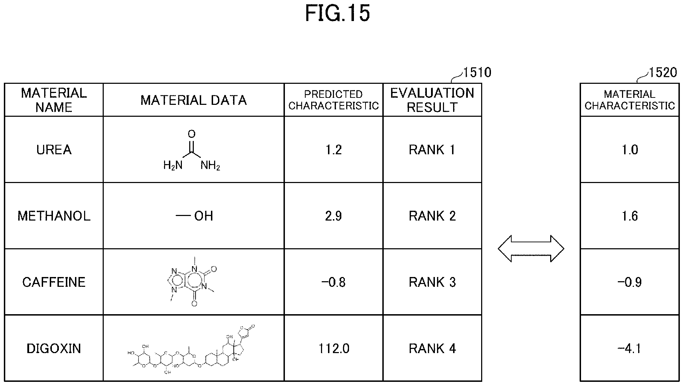

FIG. 15 is a diagram illustrating an exemplary implementation 3. As illustrated in FIG. 15, the exemplary implementation 3 predicts the predicted characteristics of a plurality of prediction target data which are:
· Urea,
· Methanol,
· Caffeine, and
· Digoxin,
and determines the section to which the attribute values were classified into, respectively, to evaluate the appropriateness (refer to a table 1510). Because the material characteristics (the water solubility also in this example) of the prediction target data described above are all known, the material characteristics are illustrated in a table 1520 for the purpose of verifying the prediction accuracy.

According to the table 1510, the relationship between the index and the evaluation result for each prediction target data is as follows:
· With respect to the prediction target data evaluated as having the rank 1 and the rank 2, the predicted characteristics with a high water solubility and a good prediction accuracy are obtained, but the structural formula is simple and the unexpectedness is low.
· With respect to the prediction target data evaluated as having the rank 3, the predicted characteristics with a relatively high water solubility and a good prediction accuracy are obtained, and the structural formula is relatively complex and the unexpectedness is high.
· With respect to the prediction target data evaluated as having the rank 4, the predicted characteristics with a high water solubility are obtained, but the material actually has a low water solubility, and the prediction accuracy is very low. The structural formula is complex and the unexpectedness is high.

### (4) Verification Example of Relationship of Index and Evaluation Result and Prediction Characteristics:

According to the exemplary implementations 1 through 3, it was found that the predicted characteristics with the high water solubility are obtained under the good prediction accuracy, and the prediction target data having the relatively complex structural formula (that is, the prediction target data having a high possibility of becoming a useful new material) is highly likely to be evaluated as having the rank 3.

Accordingly, in the following, it is further verified how high the water solubility of the prediction target data (the sucrose, the lactose, and the caffeine) evaluated as having the rank 3 in exemplary implementations 1 through 3 is (that is, whether or not the prediction target data can be regarded as objectively having the high predicted characteristics) when compared to general materials.

Specifically, the frequency distribution of the water solubility was generated based on the water solubility data set disclosed in the following Non-Patent Document, and the water solubility of the prediction target data evaluated as having the rank 3 in the exemplary implementations 1 through 3 was verified.

Non-Patent Document: J. S. Delaney, "Estimating Aqueous Solubility Directly from Molecular Structure", Journal of chemical information and computer sciences, p1000-p1005, May 24, 2004.

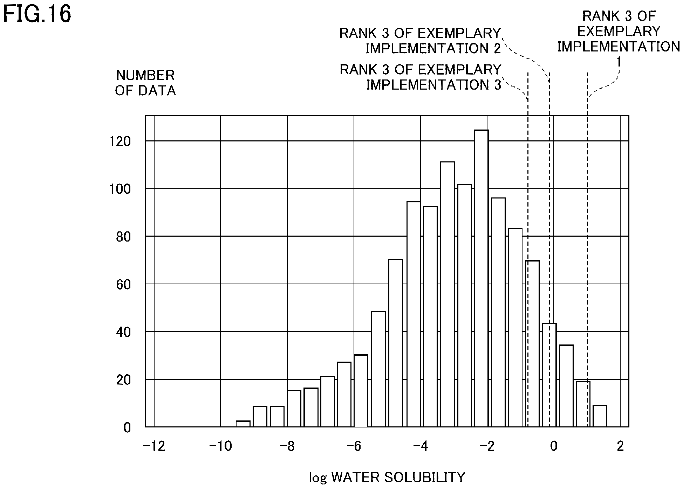

FIG. 16 is a diagram illustrating verification examples of the predicted characteristics. As illustrated in FIG. 16, it was found that the water solubility of the prediction target data evaluated as having the rank 3 in the exemplary implementations 1 through 3 is objectively high. That is, it may be regarded that the predicted characteristics of the prediction target data and the evaluation result of the prediction target data output by the prediction device 130 accurately represent the possibility of becoming a useful new material.

### <Conclusion>

As is clear from the description given heretofore, the prediction device according to the first embodiment includes:
· Acquiring the training data set used for generating the trained prediction model, and determining the plurality of sections for classifying the attribute values from the frequency distribution of the attribute values calculated between the plurality of material data included in the training data set.
· Determining the sections to which the calculated attribute values calculated between the material data of the prediction target data and the plurality of material data included in the training data set are classified into, among the plurality of sections, and evaluating the appropriateness of the prediction target data with respect to the two conflicting indexes.
· Displaying the predicted characteristics predicted by the trained model in association with the evaluation result, by inputting prediction target data to the trained model.

Hence, according to the first embodiment, even in the case where the predicted characteristics satisfying the predetermined condition are predicted using the trained prediction model, it is possible to exclude the prediction target data having a low possibility of becoming the useful new material.

As a result, the prediction device according to the first embodiment can narrow down the prediction target data for which the material generation and the verification experiment are to be performed, and can improve the development efficiency of the new material.

### [Second Embodiment]

In the first embodiment, the median, the first quartile, the third quartile, and the interquartile range are calculated as the descriptive statistics, and the coefficients α₁ and α₂ are determined to determine each section. However, the method for determining each section is not limited to the above.

For example:
· An average value of the minimum attribute values of the materials, and
· A standard deviation of the minimum attribute values of each material,
may be calculated as the descriptive statistics, and the plurality of section may be determined **as:**
· A section into which the attribute values satisfying "attribute value < (average value + β₁ × standard deviation)" are classified,
· A section into which the attribute values satisfying "(average value + β₁ × standard deviation) <= attribute value < (average value + β₂ × standard deviation)" are classified,
· A section into which the attribute values satisfying "(average value + β₂ × standard deviation) <= attribute value < (average value + β₃ × standard deviation)" are classified, and
· A section into which the attribute values satisfying "(average value + β₃ × standard deviation) <= attribute value" are classified.

In addition, although the first embodiment is described for the case where the four sections that do not overlap one another are determined, the number of sections to be determined is not limited to four, and may be three or more as long as the sections do not overlap one another.

Moreover, although the first embodiment is described for the case where one prediction target data is input to the prediction device 130 at a time, a plurality of prediction target data may be input to the prediction device 130. In this case, the prediction device 130 may be configured to display only the predicted characteristics and the evaluation result of the prediction target data having a high possibility of becoming the useful new material, for example, among the plurality of prediction target data.

Further, although the first embodiment is described for the case where the exclusion target is set, a selection target may be set, for example. Specifically, the prediction target data input unit 901 may be controlled to select the structural formula (structural formula X) of the material and input the selected structural formula to the trained prediction model 902 in a case where:
· The minimum attribute value calculated for the structural formula (structural formula X) of the material included in the "material data" of the prediction target data 700 is determined to be classified into the section (for example, the third section close to the section including the predetermined descriptive statistic) that is the selection target, or
· The structural formula (structural formula X) of the material included in the "material data" of the prediction target data 700 is evaluated as the evaluation result (for example, the rank 3) of the selection target.

In addition, although the first embodiment is described as implementing the training device and the prediction device by separate devices, the training device and the prediction device may be implemented by an integrated device.

The configuration of the present invention is not limited to the configuration or the like of the embodiments described above, and may be combined with other elements or the like. Modifications may be made without departing from the scope of the subject matter of the present invention, and the modifications can be appropriately determined according to applications.

This application is based upon and claims priority to Japanese Patent Application No. 2022-79269, filed on May 13, 2022, the entire contents of which are incorporated herein by reference.

### DESCRIPTION OF REFERENCE NUMERALS

100: Material design system
110: Material characteristic experimental device
120: Training device
130: Prediction device
140: Material design device
150: Material generation device
160: Material characteristic experimental device
201: Training data acquisition unit
202: Training unit
211: Training data attribute value calculation unit
212: Section determination unit
221: Prediction target data acquisition unit
222: Prediction unit
223: Prediction target data attribute value calculation unit
224: Evaluation unit
225: Display unit
400: Training data set
700: Prediction target data
902: Trained prediction model

## Claims

1. A prediction device comprising:
a section determination unit configured to acquire a training data set used for generating a trained prediction model, and determine a plurality of sections for classifying attribute values from a frequency distribution of the attribute values calculated between a plurality of data included in the training data set;
an evaluation unit configured to determine sections to which attribute values calculated between prediction target data and the plurality of data are classified into, among the plurality of sections, and evaluate an appropriateness of the prediction target data with respect to conflicting indexes; and
a display unit configured to display a predicted value predicted by the trained model in association with an evaluation result of the evaluation unit, by inputting the prediction target data to the trained model.

2. The prediction device as claimed in claim 1, wherein the section determination unit calculates descriptive statistics for the attribute values calculated between the plurality of data, and determines a lower limit value or an upper limit value of the attribute values that defines the plurality of sections.

3. The prediction device as claimed in claim 2, wherein the section determination unit determines three or more sections that do not overlap one another.

4. The prediction device as claimed in claim 3, wherein the evaluation unit evaluates the appropriateness of the prediction target data depending on a closeness of a section to which the attribute value calculated between the prediction target data and the plurality of data is classified into among the three or more sections, with respect to a section including a predetermined descriptive statistic.

5. The prediction device as claimed in claim 3, wherein the evaluation unit excludes the prediction target data from data to be input to the trained model when a section to which the attribute value calculated between the prediction target data and the plurality of data is classified into among the three or more sections is determined to be most distant from a section including a predetermined descriptive statistic.

6. The prediction device as claimed in claim 3, wherein the evaluation unit selects the prediction target data as the data to be input to the trained model when the attribute value calculated between the prediction target data and the plurality of data is classified into among the three or more sections is determined as a section R-th closest to a section including a predetermined descriptive statistic.

7. The prediction device as claimed in claim 1, further comprising:
a training data attribute value calculation unit configured to calculate distances among the plurality of data included in the training data set; and
a training data minimum attribute value extraction unit configured to extract a minimum distance among calculated distances between each of the plurality of data and other data,
wherein the section determination unit determines a plurality of sections for classifying the minimum distance from a frequency distribution of the extracted minimum distance.

8. The prediction device as claimed in claim 7, wherein:
the training data attribute value calculation unit calculates distances between an i-th data (1 <= i <= N) and (N-1) data excluding the i-th data among N data (N is an arbitrary integer) included in the training data set, and
the training data minimum attribute value extraction unit extracts the minimum distance from (N-1) distances calculated for the i-th data.

9. The prediction device as claimed in claim 7, further comprising:
a prediction target data attribute value calculation unit configured to calculate the distance between the prediction target data and the plurality of data; and
a prediction target data minimum attribute value extraction unit configured to extract a minimum distance among distances between the prediction target data and the plurality of data,
wherein the evaluation unit evaluates the appropriateness of the prediction target data with respect to the conflicting indexes by determining the sections to which the minimum distance extracted for the prediction target data is classified into, among the plurality of sections.

10. A material design system comprising:
the prediction device according to claim 1; and
a material design device configured to receive the prediction target data for which the evaluation unit of the prediction device determines that the attribute values calculated between the prediction target data and the plurality of data are to be classified into a predetermined section, and for which the trained model of the prediction device predicts the predicted value satisfying a predetermined condition, and generates material design data.

11. The material design system as claimed in claim 10, further comprising:
a training device configured to generate the trained model based on the training data set,
wherein the prediction device predicts the predicted value by inputting the prediction target data to the trained model generated by the training device.

12. A computer-implemented prediction method comprising the steps of:
acquiring a training data set used for generating a trained prediction model, and determining a plurality of sections for classifying attribute values from a frequency distribution of the attribute values calculated between a plurality of data included in the training data set;
determining sections to which attribute values calculated between prediction target data and the plurality of data are classified into, among the plurality of sections, and evaluating an appropriateness of the prediction target data with respect to conflicting indexes; and
displaying a predicted value predicted by the trained model in association with an evaluation result of the determining step, by inputting the prediction target data to the trained model.

13. A prediction program for causing a computer to execute the steps of:
acquiring a training data set used for generating a trained prediction model, and determining a plurality of sections for classifying attribute values from a frequency distribution of the attribute values calculated between a plurality of data included in the training data set;
determining sections to which attribute values calculated between prediction target data and the plurality of data are classified into, among the plurality of sections, and evaluating an appropriateness of the prediction target data with respect to conflicting indexes; and
displaying a predicted value predicted by the trained model in association with an evaluation result of the determining step, by inputting the prediction target data to the trained model.
